# EUROPÄISCHE PATENTSCHRIFT

(11) **EP 0 934 289 B1**
(45) Veröffentlichungstag und Bekanntmachung des Hinweises auf die Patenterteilung: **07.04.2004**
(21) Anmeldenummer: 97909363.0
(22) Anmeldetag: 06.10.1997
(51) Int. Cl.: C07D 273/00, C07D 291/02, C07D 271/06, C07D 413/00, C07D 419/00, A01N 43/72, C07C 251/40

(54) **OXIMDERIVATE UND IHRE VERWENDUNG ALS FUNGIZIDE**
OXIME DERIVATIVES AND THEIR USE AS FUNGICIDES
DERIVES D'OXIME ET LEUR UTILISATION COMME FONGICIDES

(30) Priorität: 17.10.1996 DE 19642864
(43) Veröffentlichungstag der Anmeldung: 11.08.1999
(73) Patentinhaber: Bayer CropScience AG, 40789 Monheim (DE)
(72) Erfinder: KRÜGER, Bernd-Wieland, D-51467 Bergisch Gladbach (DE); GERDES, Peter, D-52080 Aachen (DE); GAYER, Herbert, D-40789 Monheim (DE); TIEMANN, Ralf, D-51375 Leverkusen (DE); DUTZMANN, Stefan, D-40764 Langenfeld (DE); STENZEL, Klaus, D-40595 Düsseldorf (DE); HÄNSSLER, Gerd, D-51381 Leverkusen (DE)
(86) Internationale Anmeldenummer: PCT/EP1997/005475
(87) Internationale Veröffentlichungsnummer: WO 1998/017653

(56) Entgegenhaltungen:
- WO-A-95/04728
- WO-A-96/25406
- DE-A- 4 442 732

## Beschreibung

Die Erfindung betrifft neue Oximderivate, mehrere Verfahren zu ihrer Herstellung und ihre Verwendung als Fungizide, sowie neue Zwischenprodukte und mehrere Verfahren zu deren Herstellung.

Es ist bereits bekannt geworden, daß bestimmte Oximderivate, die den unten beschriebenen konstitutionell ähnlich sind, fungizide Eigenschaften besitzen (vergleiche z. B. WO-A 9504728). Die fungizide Wirkung dieser Verbindungen ist jedoch in vielen Fällen unbefriedigend.

Es wurden nun die neuen Oximderivate der allgemeinen Formel (I) gefunden, 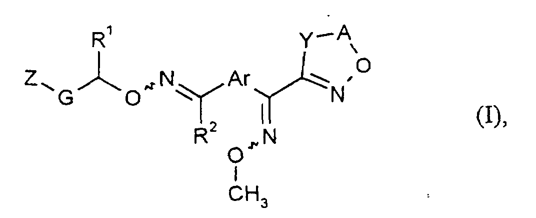 in welcher
- A: für Alkandiyl mit 1 bis 6 Kohlenstoffatomen steht,
- Ar: für jeweils gegebenenfalls substituiertes Phenylen oder Naphthylen, für mono- oder bicyclisches Heteroarylen mit jeweils 5 oder 6 Ringgliedern oder für benzokondensiertes Heteroarylen mit 5 oder 6 Ringgliedern, von denen jeweils mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
- G: für eine Einfachbindung, gegebenenfalls durch ein oder zwei Heteroatome unterbrochenes Alkandiyl mit 1 bis 5 Kettengliedern (wobei aber das Kohlenstoffatom, an das R¹ gebunden ist, stets mit einem Kohlenstoffatom der Alkandiylkette verbunden ist) oder eine Gruppierung 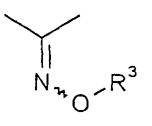 steht, worin
R³ für Alkyl mit 1 bis 4 Kohlenstoffatomen steht,
- R¹: für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht,
- R²: für Wasserstoff oder Alkyl mit 1 bis 4 Kohlenstoffatomen steht,
- Y: für Sauerstoff, Schwefel oder -NH- steht und
- Z: für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohl enstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen;
oder für jeweils gegebenenfalls einfach oder mehrfach, gleich oder verschieden substituiertes Phenyl, Naphthyl oder für Heterocyclyl mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen
Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel - oder eine Gruppierung 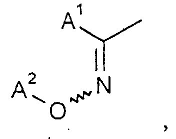
worin
- A¹: für Alkyl mit 1 bis 4 Kohlenstoffatomen oder Cycloalkyl mit 1 bis 6 Kohlenstoffatomen steht und
- A²: für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt. Ist eine Alkyl- oder Alkandiylkette durch mehr als ein Heteroatom unterbrochen, sind diese gleich oder verschieden. Ist eine Alkyl- oder Alkandiylkette durch mehr als ein Sauerstoffatom unterbrochen, stehen diese nicht direkt benachbart.

Halogen steht im allgemeinen für Fluor, Chlor, Brom oder Iod, vorzugsweise für Fluor, Chlor, oder Brom, insbesondere für Fluor oder Chlor.

Aryl steht für aromatische, mono oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind monooder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Weiterhin wurde gefunden, daß man die neuen Oximderivate der allgemeinen Formel (I) erhält, wenn man (Verfahren a)) Oxime der allgemeinen Formel (II) 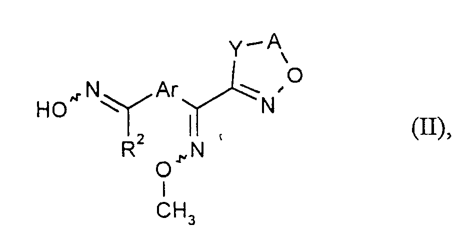 in welcher
A, Ar, Y und R² die oben angegebenen Bedeutungen haben,
mit einemAlkylierungsreagenz der allgemeinen Formel (III) 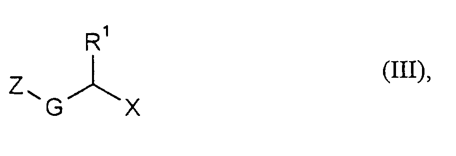 in welcher
- G, R¹ und Z: die oben angegebenen Bedeutungen haben und
- X: für Halogen, Alkylsulfonyloxy oder gegebenenfalls substituiertes Arylsulfonyloxy steht,

gegebenenfalls in Gegenwart eines Verdünnungsmittels und gegebenenfalls in Gegenwart eines Säureakzeptors umsetzt.

Schließlich wurde gefunden, daß die neuen Oximderivate der allgemeinen Formel (I) eine sehr starke fungizide Wirkung zeigen.

Die erfindungsgemäßen Verbindungen können gegebenenfalls als Mischungen verschiedener möglicher isomerer Formen, insbesondere von Stereoisomeren, wie z.B. E- und Z-, oder optischen Isomeren vorliegen. Es werden sowohl die E- als auch die Z-Isomeren, die einzelnen Enantiomeren, die Racemate, wie auch beliebige Mischungen dieser Isomeren, beansprucht.

Die vorliegende Anmeldung betrifft insbesondere Verbindungen der Formel (I), in welcher
- A: für Methylen, Ethan-1,1-diyl, Ethan-1,2-diyl, Propan-1,1-diyl, Propan-1,2-diyl, Propan-1,3-diyl, Propan-2,2-diyl, Butan-1,2-diyl, Butan-1,3-diyl oder Butan-2,3-diyl, steht,
- Ar: für jeweils gegebenenfalls einfach oder zweifach durch Fluor, Chlor, Cyano, Methyl, Ethyl, Cyclopropyl, Trifluormethyl, Methoxy, Ethoxy, Methylthio, Methylsulfinyl oder Methylsulfonyl substituiertes ortho-, meta- oder para-Phenylen, Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht,
- G: für eine Einfachbindung, Methylen, Ethan-1,2-diyl, -O-CH₂- oder eine Gruppierung 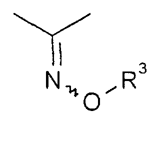 steht, worin
R³ für Methyl, Ethyl, n- oder i-Propyl steht,
- R¹: für Wasserstoff, Cyano, Methyl, Ethyl oder Cyclopropyl steht,
- R²: für Wasserstoff oder Methyl steht,
- Y: für Sauerstoff, Schwefel oder -NH- steht und
- Z: für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidyl, Pyridazinyl, Pyrazinyl, 1,2,3-Triazinyl, 1,2,4-Triazinyl, 1,3,5-Triazinyl oder 5,6-Dihydro-1,4,2-dioxazinyl steht, wobei die möglichen Substituenten vorzugsweise aus der nachstehenden Aufzählung ausgewählt sind:
Fluor, Chlor, Brom, Cyano,, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy oder eine Gruppierung 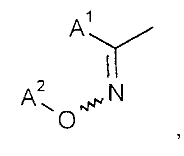 wobei
- A¹: für Methyl, Ethyl, n- oder i-Propyl, n-, i-, s- oder t-Butyl, Cyclopropyl oder Cyclobutyl steht und
- A²: für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

Eine besonders bevorzugte Gruppe erfindungsgemäßer Verbindungen sind diejenigen Verbindungen der Formel (I),
in welcher
- A: für Methylen, Ethan-1,1-diyl, Ethan-1,2-diyl, Propan-1,1-diyl, Propan-1,2-diyl, Propan-1,3-diyl, Propan-2,2-diyl, Butan-1,2-diyl, Butan-1,3-diyl oder Butan-2,3-diyl, steht,
- Ar: für ortho-Phenylen, Pyridin-2,3-diyl oder Thiophen-2,3-diyl steht,
- G: für eine Einfachbindung, Methylen, Ethan-1,2-diyl, -O-CH,- oder eine Gruppierung 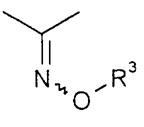 steht, worin
R³ für Methyl steht,
- R¹: für Methyl steht,
- R²: für Wasserstoff steht,
- Y: für Sauerstoff, Schwefel oder -NH- steht und
- Z: für jeweils gegebenenfalls einfach bis dreifach, gleich oder verschieden substituiertes Phenyl, Pyridyl, Pyrimidyl, Thienyl oder 5,6-Dihydro-1,4,2-dioxazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch
Fluor, Chlor, Methyl, Trifluormethyl oder Ethyl substituiertes, jeweils zweifach verknüpftes Methylendioxy oder Ethylendioxy oder eine Gruppierung 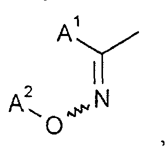 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

In einer ganz besonders bevorzugten Gruppe erfindungsgemäßer Verbindungen steht Ar für o-Phenylen.

In einer weiteren ganz besonders bevorzugten Gruppe erfindungsgemäßer Verbindungen steht Y für Sauerstoff und A für 1,2-Ethandiyl.

Die oben aufgeführten allgemeinen oder in den Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination, beliebig auch durch Restedefinitionen anderer Vorzugsbereiche ersetzt.

Beispiele für die erfindungsgemäßen Verbindungen sind in den Tabellen 1 bis 6 aufgeführt:

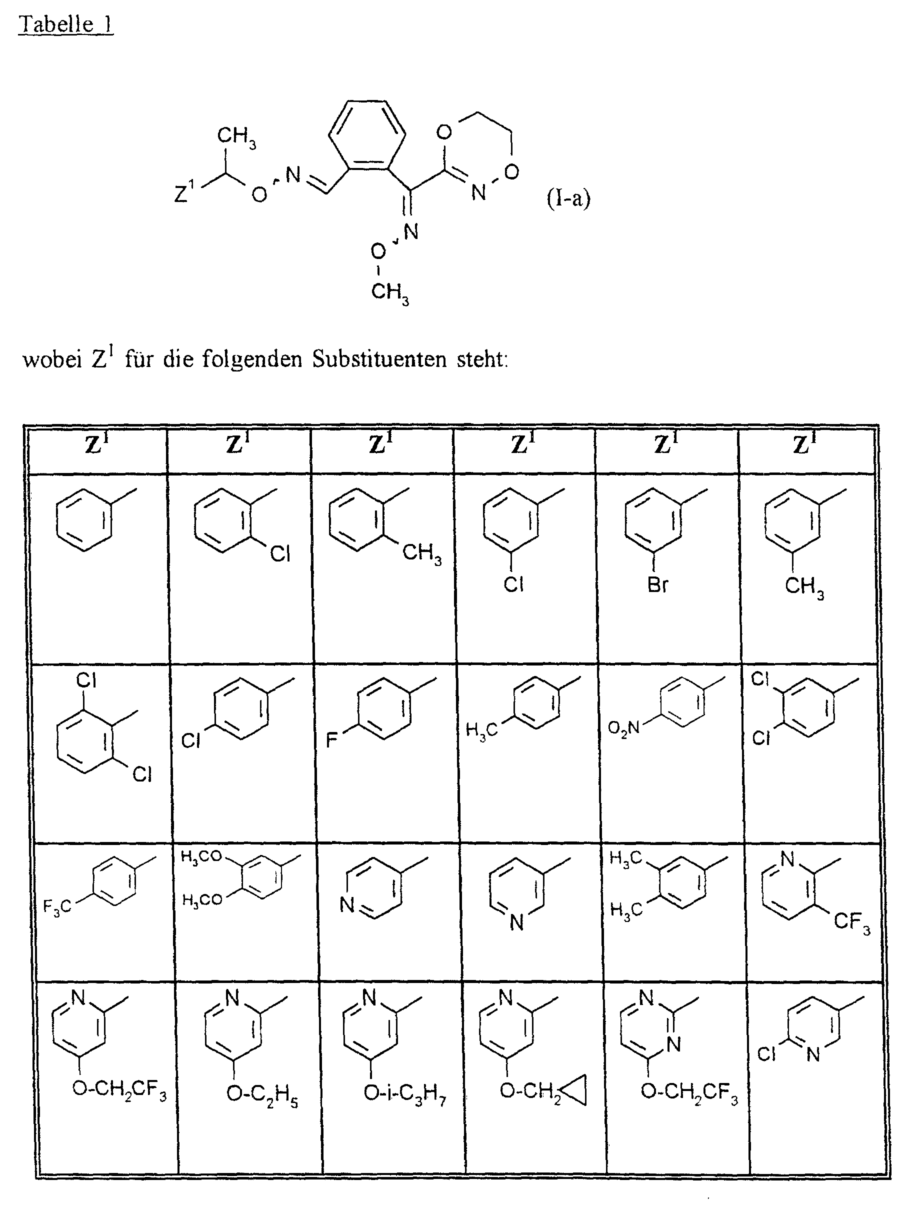

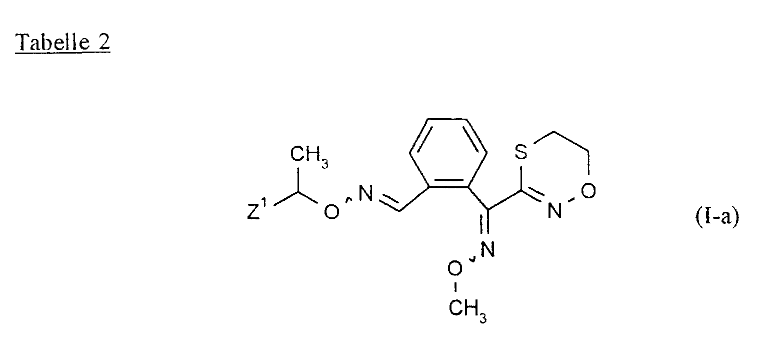

wobei Z¹ für die in Tabelle 1 genannten Substituenten steht.

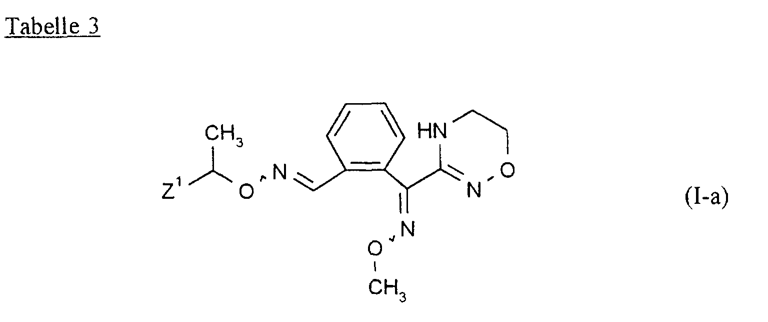

wobei Z¹ für die in Tabelle 1 genannten Substituenten steht.

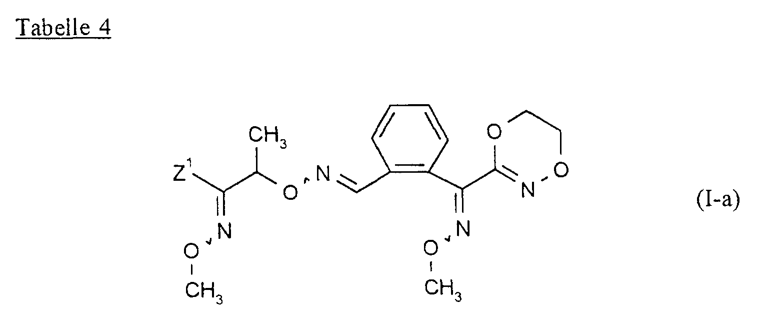

wobei Z¹ für die in Tabelle 1 genannten Substituenten steht.

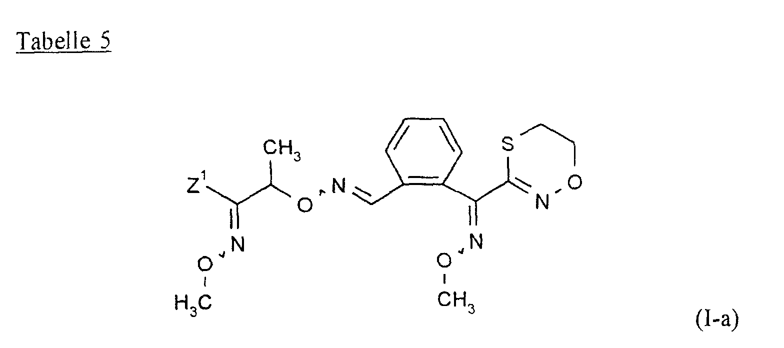

wobei Z¹ für die in Tabelle 1 genannten Substituenten steht.

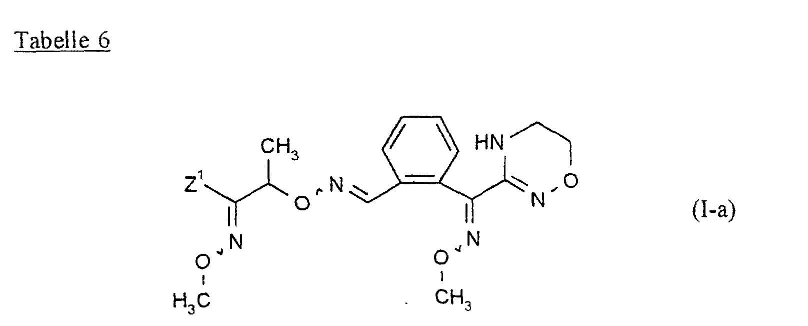

wobei Z¹ für die in Tabelle 1 genannten Substituenten steht.

Die zur Durchführung des erfindungsgemäßen Verfahrens a) als Ausgangsstoffe benötigten Oxime sind durch die Formel (II) allgemein definiert. In dieser Formel (II) haben A, Ar, Y und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A, Ar, Y und R² angegeben wurden.

Die Ausgangsstoffe der Formel (II) sind noch nicht bekannt, sie sind als neue Stoffe auch Gegenstand der vorliegenden Anmeldung.

Die Oxime der Formel (II) werden erhalten, wenn man (Verfahren b)) Ketoverbindungen der allgemeinen Formel (IV) 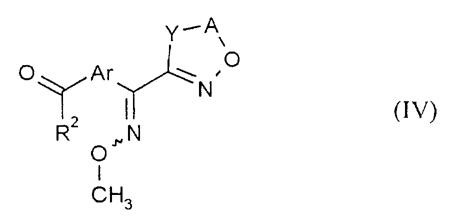 in welcher
A, Ar, Y und R² die oben angegebenen Bedeutungen haben,
mit Hydroxylamin oder einem seiner Salze,
gegebenenfalls in Gegenwart eines Verdünnungsmittels, vorzugsweise eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder reinem Wasser und gegebenenfalls in Gegenwart einer Base, vorzugsweise eines Erdalkalimetall- oder Alkalimetallhydroxids, -alkoholates, -acetates, -carbonates oder -hydrogencarbonates, wie beispielsweise Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amins, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), umsetzt.

Die zur Durchführung des erfindungsgemäßen Verfahrens b) als Ausgangsstoffe benötigten Ketoverbindungen sind durch die Formel (IV) allgemein definiert. In dieser Formel (IV) haben A, Ar, Y und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A, Ar, Y und R² angegeben wurden.

Die Ketoverbindungen der Formel (IV) sind noch nicht bekannt, sie sind als neue Stoffe ebenfalls Gegenstand der vorliegenden Anmeldung.

Die Ketoverbindungen der Formel (IV) werden erhalten, wenn man (Verfahren c)) Halogenalkylverbindungen der allgemeinen Formel (V), 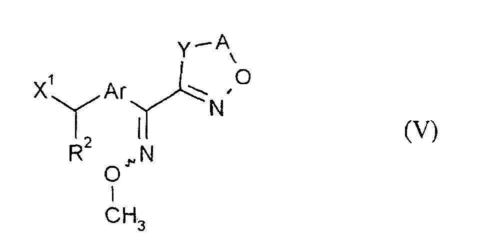 in welcher
- A, Ar, Y und R²: die oben angegebenen Bedeutungen haben,
- X¹: für Halogen steht,
nach bekannten Methoden mit Dimethylsulfoxid (Nef-Reaktion), Urotropin (Sommelet-Reaktion), einer Nitronsäure (Hass-Reaktion), einem Aminoxid oder einer Nitrosoverbindung (Kröhnke-Reaktion), gegebenenfalls in Gegenwart einer Base, vorzugsweise eines Erdalkalimetall- oder Alkalimetallhydrids, -amids, -alkoholates, wie beispielsweise Natriumhydrid, Natriumamid, Natriummethylat, Natriumethylat, Kalium-tert.-butylat und gegebenenfalls in Gegenwart eines Verdünnungsmittels, vorzugsweise eines Amids, wie N,N-Dimethylformamid oder eines Alkohols, wie Methanol, Ethanol, n- oder i-Propanol, n-, i-, sek- oder tert-Butanol, bei Temperaturen von -20 bis 150°C vorzugsweise von -10 bis 120°C, umsetzt.

Die zur Durchführung des erfindungsgemäßen Verfahrens c) als Ausgangsstoffe benötigten Halogenalkylverbindungen sind durch die Formel (V) allgemein definiert. In dieser Formel (V) haben A, Ar, Y und R² vorzugsweise bzw. insbesondere diejenigen Bedeutungen, die bereits im Zusammenhang mit der Beschreibung der erfindungsgemäßen Verbindungen der Formel (I) als bevorzugt bzw als insbesondere bevorzugt für A, Ar, Y und R² angegeben wurden. X¹ steht für Halogen, vorzugsweise für Chlor oder Brom.

Die Halogenalkylverbindungen der Formel (V) sind teilweise noch nicht bekannt.

Die Halogenalkylverbindungen der Formel (V) werden erhalten, wenn man (Verfahren (d)) Phenoxyverbindungen der allgemeinen Formel (VI), 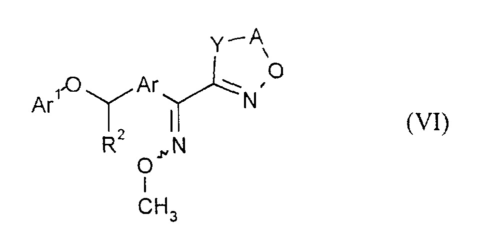 in welcher
- A, Ar, Y und R²: die oben angegebene Bedeutung hat und
- Ar¹: für gegebenenfalls substituiertes Aryl steht,
mit einem Säurehalogenid der Formel (VII),

E-X² (VII)

in welcher
- E: für Alkylcarbonyl, Arylcarbonyl, Alkylsulfonyl oder Arylsulfonyl steht und
- X²: für Halogen steht,
gegebenenfalls in Gegenwart eines Verdünnungsmittels, und gegebenenfalls in Gegenwart einer Lewis-Säure, umsetzt.

Die zur Durchführung des Verfahrens d) als Ausgangsstoffe benötigten Phenoxyverbindungen sind durch die Formel (VI) allgemein definiert. In dieser Formel (VI) haben A, Ar, Y und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A, Ar, Y und R² angegeben wurden. Ar¹ steht für gegebenenfalls substituiertes Aryl, vorzugsweise für gegebenenfalls durch Halogen, Alkyl oder Alkoxy mit jeweils 1 bis 4 Kohlenstoffatomen substituiertes Phenyl, insbesondere für Phenyl oder 2-, 3- oder 4-Methylphenyl

Die Phenoxyverbindungen der Formel (VI) sind bekannt und/oder können nach bekannten Methoden hergestellt werden (vergleiche z. B. WO-A 9 504 728 und WO-A-9 625 406).

Die zur Durchführung des Verfahrens d) weiterhin als Ausgangsstoffe benötigten Säurehalogenide sind durch die Formel (VII) allgemein definiert. In dieser Formel (VII) steht X² für Halogen, vorzugsweise für Chlor oder Brom. E steht für Alkylcarbonyl, vorzugsweise für Acetyl, Propionyl oder Pivaloyl, Arylcarbonyl, vorzugsweise für Benzoyl oder Toluyl, Alkylsulfonyl, vorzugsweise für Methylsulfonyl, oder Arylsulfonyl, vorzugsweise für Tolylsulfonyl.

Die Säurehalogenide der Formel (VII) sind allgemein bekannte Synthesechemikalien.

Die zur Durchführung des erfindungsgemäßen Verfahrens c) weiterhin benötigten Ausgangsstoffe, wie Dimethylsulfoxid, Urotropin, Nitronsäuren, Aminoxide oder Nitrosoverbindungen, sind allgemein bekannte Synthesechemikalien.

Das zur Durchführung des erfindungsgemäßen Verfahrens b) und des Verfahrens e) weiterhin als Ausgangsstoff benötigte Hydroxylamin oder dessen Salze, sind allgemein bekannte Synthesechemikalien.

Die zur Durchführung des erfindungsgemäßen Verfahrens a) weiterhin als Ausgangsstoffe benötigten Alkylierungsreagenzien sind durch die Formel (III) allgemein definiert. In der Formel (III) haben G und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für G und Z angegeben wurden. X steht für Halogen, vorzugsweise für Chlor, Brom, oder Iod, oder für Alkylsulfonyl, vorzugsweise Methylsulfonyl, oder für gegebenenfalls substituiertes Arylsulfonyl, vorzugsweise 4-Tolylsulfonyl.

Die Alkylierungsreagenzien der allgemeinen Formel (III) sind teilweise bekannt und nach bekannten Verfahren herstellbar. (vergleiche z. B. J.Org.Chem., 51, l, 1986, 109-111; J.Chem.Soc.Perkin Trans.2, 1986, 593-598).

Neu sind Alkylierungsreagenzien der allgemeinen Formel (III-a), 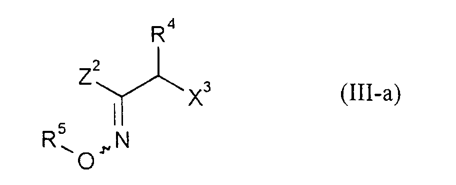 in welcher
- R⁴: für Alkyl mit 1 bis 4 Kohlenstoffatomen oder Cycloalkyl mit 3 bis 6 Kohlenstoffatomen steht,
- R⁵: für Alkyl mit 1 bis 4 Kohlenstoffatomen steht,
- X³: für Chlor, Brom oder Iod steht und
- Z²: für jeweils gegebenenfalls einfach oder mehrfach, gleich oder verschieden substituiertes Phenyl, Naphthyl oder für Heterocyclyl mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel - oder eine Gruppierung 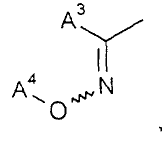 worin
- A³: für Alkyl mit 1 bis 4 Kohlenstoffatomen oder Cycloalkyl mit 1 bis 6 Kohlenstoffatomen steht und
- A⁴: für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht.

Besonders bevorzugt sind Verbindungen der Formel (III-a), in welcher
- R⁴: für Methyl, Ethyl oder Cyclopropyl steht,
- R⁵: für Methyl oder Ethyl steht,
- X³: für Chlor oder Brom steht und
- Z²: für jeweils gegebenenfalls einfach bis dreifach, gleich oder verschieden substituiertes Phenyl, Pyridyl, Pyrimidyl oder Thienyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy oder eine Gruppierung 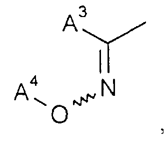 worin
- A³: für Methyl, Ethyl, n- oder i-Propyl, n-, i-, s- oder t-Butyl, Cyclopropyl oder Cyclobutyl steht und
- A⁴: für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

Die Alkylierungsreagenzien der Formel (III-a) werden erhalten (Verfahren e)), wenn man Halogenketone der allgemeinen Formel (VIII), 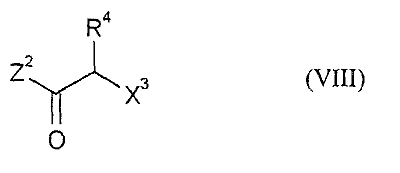 in welcher
R⁴, X³ und Z² die oben angegebenen Bedeutungen haben,
mit Hydroxylamin oder einem seiner Salze,
gegebenenfalls in Gegenwart eines Verdünnungsmittels, vorzugsweise eines eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder reinem Wasser und gegebenenfalls in Gegenwart einer Base, vorzugsweise eines Erdalkalimetall- oder Alkalimetallhydroxids, -alkoholates, -acetates, -carbonates oder -hydrogencarbonates, wie beispielsweise Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amins, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), umsetzt.

Die zur Durchführung des Verfahrens e) als Ausgangsstoffe benötigten Halogenketone sind durch die Formel (III) allgemein definiert. In der Formel (III) haben R⁴, X³ und Z² vorzugsweise bzw. insbesondere diejenigen Bedeutungen, die bereits im Zusammenhang mit der Beschreibung der erfindungsgemäßen Verbindungen der Formel (III-a) als bevorzugt bzw. als insbesondere bevorzugt für R⁴, X³ und Z² angegeben wurden.

Die zur Durchführung des Verfahrens e) als Ausgangsstoffe benötigte Halogenketone der Formel (VIII) sind allgemein bekannte Synthesechemikalien.

Als Verdünnungsmittel zur Durchführung des erfindungsgemäßen Verfahrens a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n-oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid, oder Sulfone, wie Sulfolan.

Das erfindungsgemäße Verfahren a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens a) in einem größeren Bereich variiert werden. Im allgemeinen arbeitet man bei Temperaturen von -20°C bis 150°C, vorzugsweise bei Temperaturen von 0°C bis 80°C.

Zur Durchführung des erfindungsgemäßen Verfahrens a) zur Herstellung der Verbindungen der Formel (I) setzt man pro Mol des Oxims der Formel (II) im allgemeinen 1 bis 15 Mol, vorzugsweise 1 bis 8 Mol Alkylierungsreagenz der Formel (III) ein.

Die erfindungsgemäßen Verfahren a), b) und c), sowie die Verfahren d) und e) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakteria, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Psdeudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, von Krankheiten im Wein-, Obst- und Gemüseanbau, wie beispielsweise gegen Venturia-, Sphaerotheca-, Podosphaera-, Phytophtora- und Plasmopara-Arten, oder von Reiskrankheiten, wie beispielsweise gegen Pyricularia-Arten, einsetzen. Mit gutem Erfolg werden auch Getreidekrankheiten, wie beispielsweise Septoria-, Pyrenophora- oder Cochliobolus-Arten, bekämpft. Ferner lassen sich die erfindungsgemäßen Verbindungen auch zur Steigerung des Ernteertrags von Kulturpflanzen einsetzen.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetate, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol, Ofurace, Oxadixyl, Oxamocarb, Oxolinicacid, Oxycarboxim, Oxyfenthiin, Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methy)-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)ethyl)amino]carbonyl]propyl}-carbaminsäure-1-isopropylester
1-(2,4-Dichlorphenyl)-2-(1H-1,2,4-triazol-1-yl)ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)methoxy]phenyl]ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[ 1-(4-chlorphenyl)ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)phenyl]methyl]-benzamid,
2-(2,3,3-Triiod-2-propenyl)-2H-tetrazol,
2-[(1-Methylethyl)sulfonyl]-5-(trichlormethyl)-1,3,4-thiadiazol,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)oxy]methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin, 8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol, -Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis(2-propinyloxy)-phenyl]-N'-methoxy-methanimidamid,
N-Formyl-N-hydroxy-DL-alanin, --Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin, Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb, HCH, Heptenophos, Hexaflumuron, Hexythiazox,
Imidacloprid, Iprobenfos, Isazophos, Isofenphos, Isoprocarb, Isoxathion, Ivermectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich,

Die Wirkstoffe können als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw.. Es ist ferner möglich die Wirkstoffe nach dem Ultra-Low-Volume-Verfahren auszubringen oder die Wirkstoffzubereitung oder der Wirkstoff selbst in den Boden zu injizieren. Es kann wird auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1

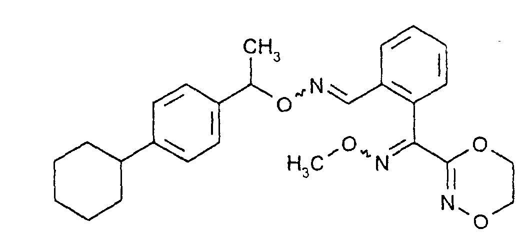

Eine Lösung von 0,5 g (1,9 mMol) 2-[(5,6-Dihydro-[1,4,2]dioxazin-3-yl)-methoxyimino-methyl]-benzaldehydoxim in 10 ml Dimethylformamid wird auf 0°C gekühlt und unter Argon mit 0,063 g (2,1 mMol) 80%iger Natriumhydridsuspension versetzt. Zu dieser Mischung tropft man eine Lösung von 0,42 g (2,1 mMol) 1-(1-Chlorethyl)-4-cyclohexylbenzol in 2 ml Dimethylformamid und rührt 4 Stunden ohne weitere Kühlung. Man gießt die Reaktionsmischung auf 150 ml Wasser und extrahiert 3 mal mit jeweils 100 ml Essigsäureethylester. Die vereinigten organischen Phasen werden über Magnesiumsulfat getrocknet und bei vermindertem Druck eingeengt. Der Rückstand wird mit Cyclohexan/Essigester (3:1) an Kieselgel chromatografiert. Man erhält 0,6 g (70 % der Theorie) 2-[(5,6-Dihydro[1,4,2]dioxazin-3-yl)-methoxyimino-methyl]-benzaldehyd-O-[1-(4-cyclohexylphenyl)-ethyl]-oxim.
- ¹H-NMR (CDCl3, TMS):: δ = 1,20-1,40; 1,56; 1,59; 1,70-1,90; 2,48; 3,92;
4,07-4,16; 4,42-4,49; 5,30; 7,13-8,00 ppm.

### Herstellung des Ausgangsstoffes

### Beispiel II-1

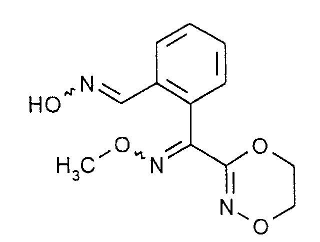

Eine Lösung von 4 g (0,16 mol) 2-[(5,6-Dihydro-[1,4,2]dioxazin-3-yl)-methoxyimino-methyl]-benzaldehyd in 100 ml Ethanol wird unter Argon bei 20°C mit 5,5 g (0,08 Mol) Hydroxylammoniumchlorid und 8 g (0,08 Mol) Triethylamin versetzt und 2 Stunden bei 20°C gerührt. Die Mischung wird auf 500 ml Wasser gegossen und dreimal mit jeweils 100 ml Dichlormethan extrahiert. Die vereinigten organischen Phasen werden über Magnesiumsulfat getrocknet und bei vermindertem Druck eingeengt. Der Rückstand wird mit Cyclohexan/Essigester (4:1) an Kieselgel chromatografiert. Man erhält 2,4 g (57 % der Theorie) 2-[(5,6-Dihydro-[1,4,2]dioxazin-3-yl)-methoxyimino-methyl]-benzaldehydoxim.
- ¹H-NMR (CDCl3, TMS):: δ = 3,97; 4,14-4,19; 4,49-4,52; 7,18-8,03 ppm.

### Herstellung der Vorstufe

### Beispiel IV-1

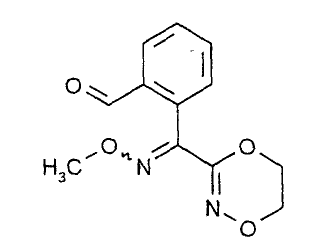

Zu einer Lösung von 15 g (0,056 Mol) (2-Chlormethyl-phenyl)-(5,6-dihydro[1,4,2]dioxazin-3-yl)-methanon-O-methyl-oxim in 150 ml Dimethylformamid gibt man 19,5 g (0,14 Mol) N-Methylmorpholin-N-oxid und rührt 2 Stunden bei 120°C. Nach dem Abkühlen wird die Mischung in 1000 ml Wasser gegossen und drei mal mit jeweils 200 ml Essigsäureethylester extrahiert. Die vereinigten organischen Phasen werden über Magnesiumsulfat getrocknet und bei vermindertem Druck eingeengt. Der Rückstand wird mit Diisopropylether verrührt und der entstandene Feststoff abgesaugt. Man erhält 10,5 g (76 % der Theorie) 2-[(5,6-Dihydro-[1,4,2]dioxazin-3-yl)-methoxyimino-methyl]-benzaldehyd.
- ¹H-NMR (CDCl3, TMS):: δ = 3,96; 4,17-4,20; 4,53-4,56; 7,29-7,95; 9,92 ppm.

### Herstellung der Vorstufe

### Beispiel V-1

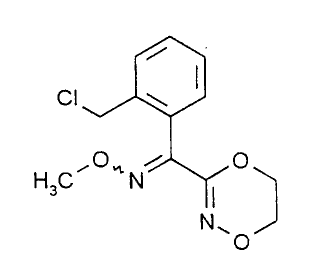

Zu einer Suspension von 103,4 g (0,775 Mol) wasserfreiem Aluminiumchlorid in 1 l Dichlormethan gibt man innerhalb von 15 Minuten 61,1 g (0,775 Mol) Acetylchlorid zu. Zu dieser Mischung tropft man unter Argon bei 20°C eine Lösung von 105 g (0,31 Mol) KBR5896 in 500 ml Dichlormethan zu, wobei sich die Reaktionsmischung bis auf 30°C erwärmt und rührt noch weitere 3 Stunden. Die Reaktionsmischung wird auf 2 l Eiswasser gegossen und 3 mal mit jeweils 300 ml Dichlormethan extrahiert. Die vereinigten organischen Phasen werden über Magnesiumsulfat getrocknet und bei vermindertem Druck eingeengt. Der Rückstand wird mit Diisopropylether verrührt und der entstandene Feststoff abgesaugt (59,1 g). Das Filtrat wird bei vermindertem Druck eingeengt und der Rückstand mit Cyclohexan/Essigester (3:1) an Kieselgel chromatografiert. Man erhält weitere 4 g Produkt. Insgesamt erhält man 63,1 g (76 % der Theorie) (2-Chlormethyl-phenyl)-(5,6-dihydro-[1,4,2]dioxazin-3-yl)-methanon-O-methyl-oxim.
- ¹H-NMR (CDCl3, TMS):: δ = 3,99; 4,17-4,20; 4,49-4,53; 7,15-7,53 ppm.

### Beispiel (2)

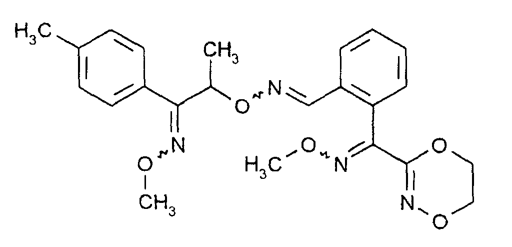

Eine Lösung von 0,4 g (1,52 mMol) 2-[(5,6-Dihydro-[1,4,2]dioxazin-3-yl)-methoxyimino-methyl]-benzaldehydoxim in 10 ml Dimethylformamid wird auf 0°C gekühlt und unter Argon mit 0,055 g (1,82 mMol) 80%iger Natriumhydridsuspension versetzt. Zu dieser Mischung tropft man eine Lösung von 0,37 g (1,52 mMol) 2-Brom-1-(p-tolyl)-propan-1-on-O-methyl-oxim in 2 ml Dimethylformamid und rührt 4 Stunden ohne weitere Kühlung. Man gießt die Reaktionsmischung auf 100 ml Wasser und extrahiert 3 mal mit jeweils 100 ml Essigsäureethylester. Die vereinigten organischen Phasen werden über Magnesiumsulfat getrocknet und bei vermindertem Druck eingeengt. Der Rückstand wird mit Cyclohexan/Essigester (3:1) an Kieselgel chromatografiert. Man erhält 0,42 g (63 % der Theorie) 2-[(5,6-Dihydro-[1,4,2]dioxazin-3-yl)-methoxyimino-methyl]-benzaldehyd- O-[2-methoxyimino-1-methyl-2-(p-tolyl)-ethyl]-oxim.
- ¹H-NMR (CDCl3, TMS):: δ = 1,56; 1,59; 2,32; 3,87; 3,99; 4,16; 4,48; 5,87;
7,11-7,98 ppm.

### Herstellung des Ausgangsstoffes

### Beispiel (III-a-1)

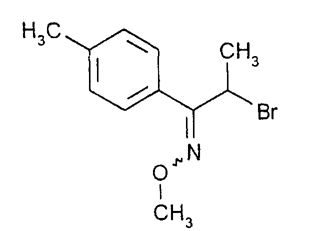

2,27 (10 mMol) 2-Brom-1-(p-tolyl)-propan-1-on werden in 10 ml Essigsäure gelöst und bei 20°C mit 1,25 g (15 mMol) Hydroxylammoniumchlorid und 2,07 g. (15 mMol) Natriumacetat versetzt. Nach 14 Stunden wird das Lösungsmittel bei vermindertem Druck abdestilliert, der Rückstand in 100 ml Essigsäureethylester aufgenommen, die organische Phase abgetrennt, über 50 g Magnesiumsulfat getrocknet und eingeengt. Man erhält 2,5 g (99 % der Theorie) 2-Brom-1-(p-tolyl)-propan-1-on-O-methyl-oxim als Isomerengemisch (e/z = 70/30).
- ¹H-NMR (DMSO-d₆, TMS):: δ = 1,69 (d, 3H, e-Isomer); 1,82 (d, 3H, z-Isomer); 3,78 (s, 3H, z-Isomer); 3,96 (s, 3H, e-Isomer) ppm.

Analog Beispiel (1), sowie entsprechend der allgemeinen Beschreibung des erfindungsgemäßen Herstellungsverfahrens a) erhält man auch die in der nachstehenden Tabelle 7 aufgeführten erfindungsgemäßen Verbindungen der Formel (I-a):

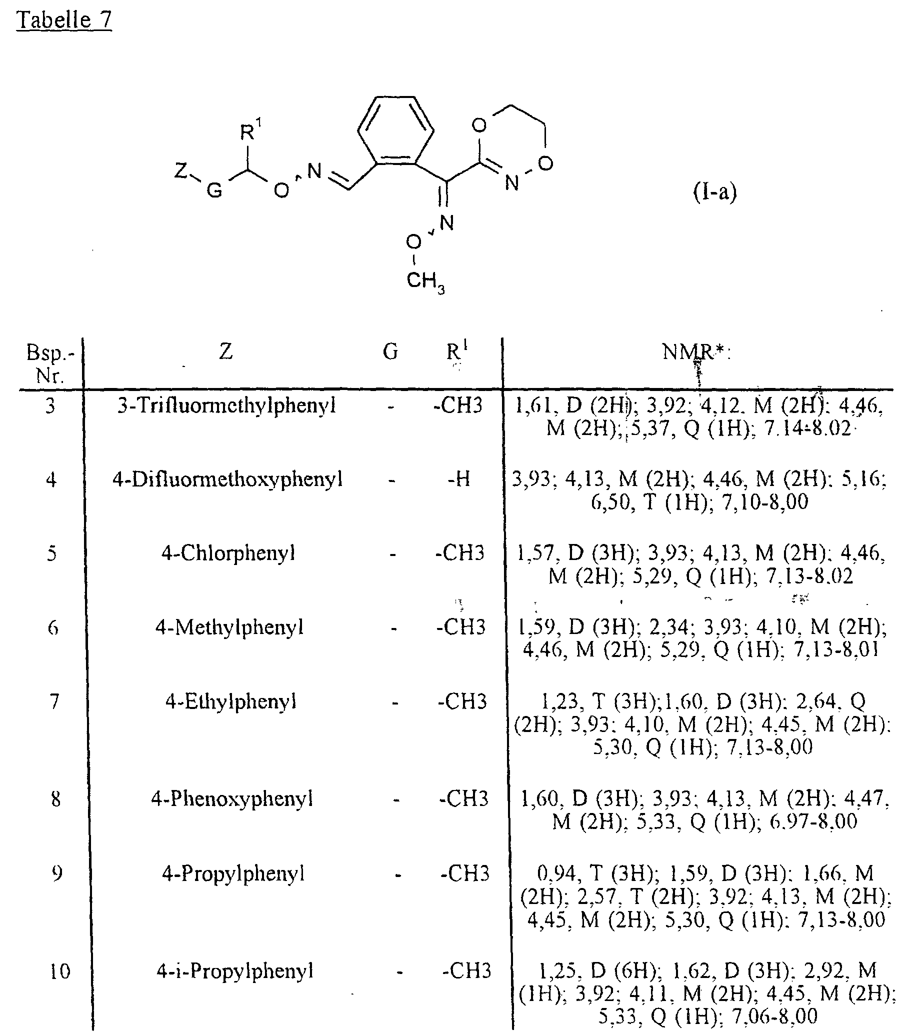

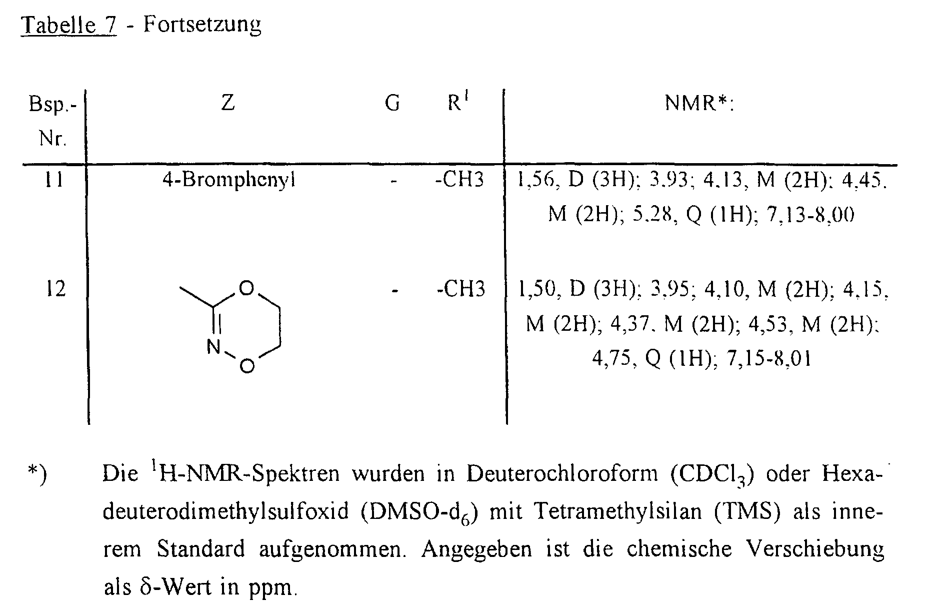

Analog Beispiel (III-a-1), sowie entsprechend der allgemeinen Beschreibung des Herstellungsverfahrens e) erhält man auch die in der nachstehenden Tabelle 8 aufgeführten Verbindungen der Formel (III-a):

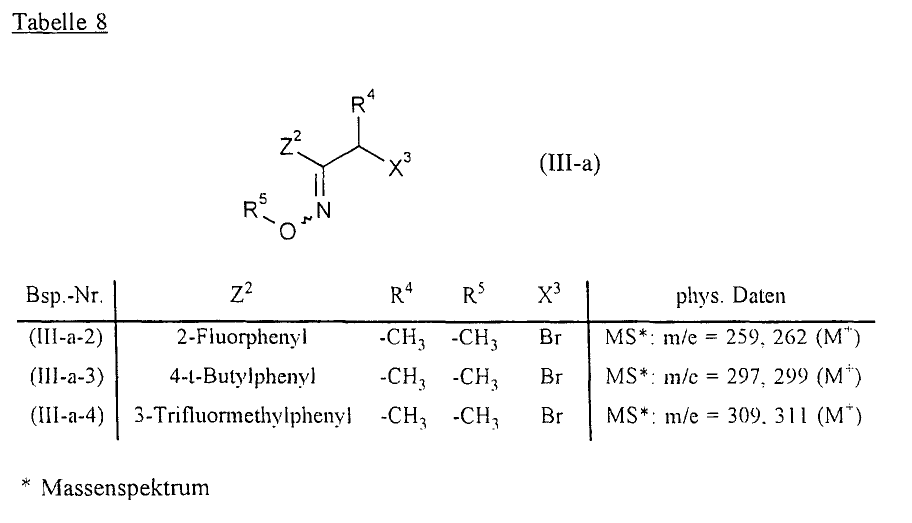

### Anwendungsbeispiele

### Beispiel 1

### Pyricularia-Test (Reis) / protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Antrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung.

Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigen z.B. die folgenden Verbindungen der Herstellungsbeispiele 5, 6, 7, 8, 9, 10 und 11 bei einer beispielhaften Wirkstoffkonzentration von 0,05% einen Wirkungsgrad von 100 %. Die Verbindung des Herstellungsbeispieles 1 zeigte bei der gleichen Wirkstoffkonzentration einen Wirkungsgrad von 90%.

### Beispiel 2

### Phytophthora-Test (Tomate) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert.

Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigen z.B. die folgenden Verbindungen der Herstellungsbeispiele 9, 10 und 11 bei einer beispielhaften Wirkstoffaufwandmenge von 100 g/ha einen Wirkungsgrad von 91-93 % gegenüber der unbehandelten Kontrolle.

### Beispiel 3

### Plasmopara-Test (Reben) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei 20°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigen z.B. die folgenden Verbindungen der Herstellungsbeispiele 5, 6, 7, 8, 9, 10 und 11 bei einer beispielhaften Wirkstoffaufwandmenge von 100 g/ha einen Wirkungsgrad von 99-100 % im Vergleich zur unbehandelten Kontrolle.

### Beispiel 4

### Podosphaera-Test (Apfel) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigen z.B. die folgenden Verbindungen der Herstellungsbeispiele 3 und 9 bei einer beispielhaften Wirkstoffaufwandmenge von 10 g/ha einen Wirkungsgrad von 94-98 % im Vergleich zur unbehandelten Kontrolle.

### Beispiel 5

### Sphaerotheca-Test (Gurke) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Sphaerotheca fuliginea inokuliert. Die Pflanzen werden dann im Gewächshaus bei ca. 23°C und einer relativen Luftfeuchtigkeit von ca. 70 % im Gewächshaus aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigen z.B. die folgenden Verbindungen der Herstellungsbeispiele 2, 3, 5, 6, 7, 9, 10 und 11 bei einer beispielhaften Wirkstoffaufwandmenge von 100 g/ha einen Wirkungsgrad von 92-100 % im Vergleich zur unbehandelten Kontrolle.

### Beispiel 6

### Venturia-Test (Apfel) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigen z.B. die folgenden Verbindungen der Herstellungsbeispiele 1, 3, 5, 6, 7, 9, 10 und 11 bei einer beispielhaften Wirkstoffaufwandmenge von 100 g/ha einen Wirkungsgrad von 94-100 % im Vergleich zur unbehandelten Kontrolle.

### Beispiel 7

### Erysiphe-Test (Gerste) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigen z.B. die folgenden Verbindungen der Herstellungsbeispiele 1, 6, 7, 9, 10 und 11 bei einer beispielhaften Wirkstoffaufwandmenge von 250 g/ha einen Wirkungsgrad von 88 % im Vergleich zur unbehandelten Kontrolle.

## Patentansprüche

1. Verbindungen der Formel (I), 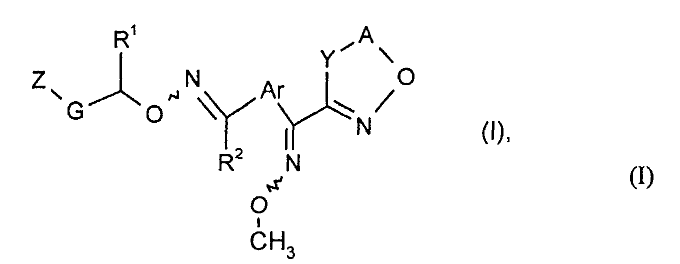 in welcher
A für Alkandiyl mit 1 bis 6 Kohlenstoffatomen steht,
Ar für jeweils gegebenenfalls substituiertes Phenylen oder Naphthylen, für mono- oder bicyclisches Heteroarylen mit jeweils 5 oder 6 Ringgliedern oder für benzokondensiertes Heteroarylen mit 5 oder 6 Ringgliedem, von denen jeweils mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/ oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
G für eine Einfachbindung, gegebenenfalls durch ein oder zwei Heteroatome unterbrochenes Alkandiyl mit 1 bis 5 Kettengliedern (wobei aber das Kohlenstoffatom, an das R' gebunden ist, stets mit einem Kohlenstoffatom der Alkandiylkette verbunden ist) oder eine Gruppierung 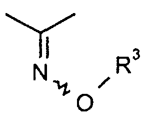 steht, worin
R³ für Alkyl mit 1 bis 4 Kohlenstoffatomen steht,
R' für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht,
R² für Wasserstoff oder Alkyl mit 1 bis 4 Kohlenstoffatomen steht,
Y für Sauerstoff, Schwefel oder -NH- steht und
Z für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen;
oder für jeweils gegebenenfalls einfach oder mehrfach, gleich oder verschieden substituiertes Phenyl, Naphthyl oder für Heterocyclyl mit 3 bis 7 Ringgliedem, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel - oder eine Gruppierung 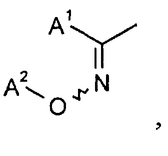 worin
A¹ für Alkyl mit 1 bis 4 Kohlenstoffatomen oder Cycloalkyl mit 1 bis 6 Kohlenstoffatomen steht und
A² für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht.

2. Verbindungen der Formel (I) gemäß Anspruch 1, in welcher
A für Methylen, Ethan-1,1-diyl, Ethan-1,2-diyl, Propan-1,1-diyl, Propan1,2-diyl, Propan-1,3-diyl, Propan-2,2-diyl, Butan-1,2-diyl, Butan-1,3-diyl oder Butan-2,3-diyl, steht,
Ar für jeweils gegebenenfalls einfach oder zweifach durch Fluor, Chlor, Cyano, Methyl, Ethyl, Cyclopropyl, Trifluormethyl, Methoxy, Ethoxy, Methylthio, Methylsulfinyl oder Methylsulfonyl substituiertes ortho-, meta- oder para-Phenylen, Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht,
G für eine Einfachbindung, Methylen, Ethan-1,2-diyl, -O-CH₂- oder eine Gruppierung 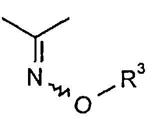 steht, worin
R³ für Methyl, Ethyl, n- oder i-Propyl steht,
R¹ für Wasserstoff, Cyano, Methyl, Ethyl oder Cyclopropyl steht,
R² für Wasserstoff oder Methyl steht,
Y für Sauerstoff, Schwefel oder -NH- steht und
Z für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidyl, Pyridazinyl, Pyrazinyl, 1,2,3-Triazinyl, 1,2,4-Triazinyl, 1,3,5-Triazinyl oder 5,6-Dihydro-1,4,2-dioxazinyl steht, wobei die möglichen Substituenten vorzugsweise aus der nachstehenden Aufzählung ausgewählt sind:
Fluor, Chlor, Brom, Cyano,, n-, i-, s- oder t-Butyl, Methoxy, Ethoxy, n-oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy oder eine Gruppierung 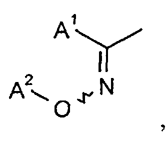 wobei
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

3. Verbindungen der Formel (I) gemäß Anspruch 1,
in welcher
A für Methylen, Ethan-1,1-diyl, Ethan-1,2-diyl, Propan-1,1-diyl, Propan-1,2-diyl, Propan-1,3-diyl, Propan-2,2-diyl, Butan-1,2-diyl, Butan-1,3-diyl oder Butan-2,3-diyl, steht,
Ar für ortho-Phenylen, Pyridin-2,3-diyl oder Thiophen-2,3-diyl steht,
G für eine Einfachbindung, Methylen, Ethan-1,2-diyl, -O-CH₂- oder eine Gruppierung 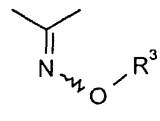 steht, worin
R³ für Methyl steht,
R¹ für Methyl steht,
R² für Wasserstoff steht,
Y für Sauerstoff, Schwefel oder -NH- steht und
Z für jeweils gegebenenfalls einfach bis dreifach, gleich oder verschieden substituiertes Phenyl, Pyridyl, Pyrimidyl, Thienyl oder 5,6-Dihydro-1,4,2-dioxazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy oder eine Gruppierung 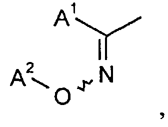 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

4. Schädlingsbekämpfungsmittel, **gekennzeichnet durch** einen Gehalt an mindestens einer Verbindung der Formel (I) nach Anspruch 1.

5. Verfahren zur Bekämpfung von Schädlingen, **dadurch gekennzeichnet, dass** man Verbindungen der Formel (I) nach Anspruch 1 auf Schädlinge und/oder ihren Lebensraum einwirken lässt.

6. Verfahren zur Herstellung von Verbindungen der Formel (I) wie in Anspruch 1 definiert, **dadurch gekennzeichnet, dass** Oxime der allgemeinen Formel (II), 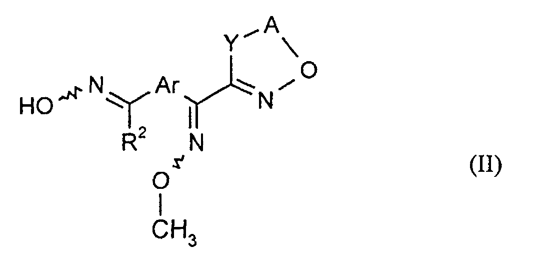 in welcher
A, Ar, Y und R² die in Anspruch 1 angegebenen Bedeutungen haben,
mit einem Alkylierungsreagenz der allgemeinen Formel (III), 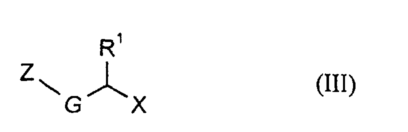 in welcher
G, R¹ und Z die oben angegebenen Bedeutungen haben und
X für Halogen, Alkylsulfonyloxy oder gegebenenfalls substituiertes Arylsulfonyloxy steht,
gegebenenfalls in Gegenwart eines Verdünnungsmittels und gegebenenfalls in Gegenwart eines Säureakzeptors umsetzt.

7. Verwendung von Verbindungen der Formel (I) nach den Ansprüchen 1 bis 3 zur Bekämpfung von Schädlingen.

8. Verfahren zur Herstellung von Schädlingsbekämpfungsmitteln, **dadurch gekennzeichnet, dass** man Verbindungen der Formel (I) nach den Ansprüchen 1 bis 3 mit Streckmitteln und/oder oberflächenaktiven Mitteln vermischt.

9. Verbindungen der Formel (II), 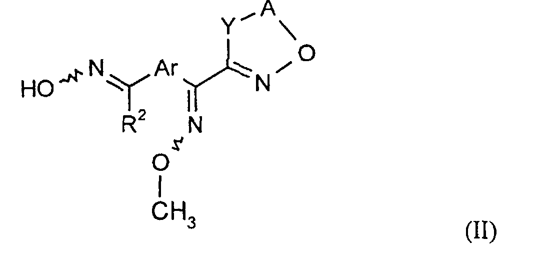 in welcher
R², Ar, Y und A die in Anspruch 1 angegebenen Bedeutungen haben.

10. Verbindungen der Formel (IV) 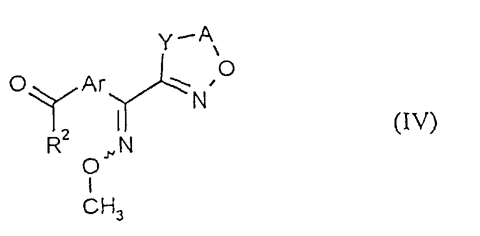 in welcher
R², Ar, Y und A die in Anspruch 1 angegebenen Bedeutungen haben.

## Claims

1. Compounds of the formula (I) in which
A represents alkanediyl having 1 to 6 carbon atoms,
Ar represents in each case optionally substituted phenylene or naphthylene, represents mono- or bicyclic heteroarylene having in each case 5 or 6 ring members or represents benzo-fused heteroarylene having 5 or 6 ring members at least one of which in each case represents oxygen, sulphur or nitrogen and one or two others optionally represent nitrogen, where the possible substituents are preferably selected from the list below:
halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl having in each case 1 to 6 carbon atoms, in each case straight-chain or branched alkenyl, alkenyloxy or alkinyloxy having in each case 2 to 6 carbon atoms, in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms, in each case straight-chain or branched halogenoalkenyl or halogenoalkenyloxy having in each case 2 to 6 carbon atoms and 1 to 11 identical or different halogen atoms, in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties, in each case doubly attached alkylene or dioxyalkylene having in each case 1 to 6 carbon atoms and being in each case optionally mono- or polysubstituted by identical or different substituents selected from the group consisting of halogen, straight-chain or branched alkyl having 1 to 4 carbon atoms and straight-chain or branched halogenoalkyl having 1 to 4 carbon atoms and 1 to 9 identical or different halogen atoms,
G represents a single bond, represents alkanediyl having 1 to 5 chain members which is optionally interrupted by one or two heteroatoms (but where the carbon atom to which R¹ is attached is in each case linked to a carbon atom of the alkanediyl chain) or a grouping
in which 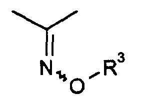
R³ represents alkyl having 1 to 4 carbon atoms,
R¹ represents hydrogen, cyano, represents in each case optionally halogen-, cyano- or C₁-C₄-alkoxy-substituted alkyl, alkoxy, alkylthio, alkylamino or dialkylamino having in each case 1 to 6 carbon atoms in the alkyl groups or represents in each case optionally halogen-, cyano-, carboxyl-, C₁-C₄-alkyl- or C₁-C₄-alkoxy-carbonyl-substituted cycloalkyl having 3 to 6 carbon atoms,
R² represents hydrogen or alkyl having 1 to 4 carbon atoms,
Y represents oxygen, sulphur or -NH- and
Z represents alkyl having 1 to 8 carbon atoms which is optionally mono-or polysubstituted by an identical or different substituent selected from the group consisting of halogen, cyano, hydroxyl, amino, C₁-C₄-alkoxy, C₁-C₄-alkylthio, C₁-C₄-alkylsulphinyl and C₁-C₄-alkylsulphonyl (each of which may optionally be substituted by halogen);
represents in each case optionally halogen-substituted alkenyl or alkinyl having in each case up to 8 carbon atoms;
represents cycloalkyl having 3 to 6 carbon atoms which is in each case optionally mono- or polysubstituted by identical or different substituents selected from the group consisting of halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl and C₁-C₄-alkoxy-carbonyl;
or represents phenyl, naphthyl or each of which radicals is optionally mono- or polysubstituted by identical or different substituents, heterocyclyl having 3 to 7 ring members, at least one of which represents oxygen, sulphur or nitrogen and one or two others optionally represent nitrogen, where the possible substituents are preferably selected from the list below:
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl or alkylsulphonyloxy having in each case 1 to 6 carbon atoms in the individual alkyl moieties;
in each case doubly attached alkylene or dioxyalkylene having in each case 1 to 6 carbon atoms and being in each case optionally mono- or polysubstituted by identical or different substituents selected from the group consisting of halogen, straight-chain or branched alkyl having 1 to 4 carbon atoms and straight-chain or branched halogenoalkyl having from 1 to 4 carbon atoms and 1 to 9 identical or different halogen atoms;
cycloalkyl having 3 to 6 carbon atoms;
heterocyclyl or heterocyclyl-methyl having in each case 3 to 7 ring members, 1 to 3 of which are in each case identical or different heteroatoms - in particular nitrogen, oxygen and/or sulphur - or a grouping 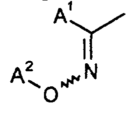 in which
A¹ represents alkyl having 1 to 4 carbon atoms or cycloalkyl having 1 to 6 carbon atoms and
A² represents optionally cyano-, alkoxy-, alkylthio-, alkylamino-, dialkylamino- or phenyl-substituted alkyl having 1 to 4 carbon atoms, alkenyl or alkinyl having in each case 2 to 4 carbon atoms.

2. Compounds of the formula (I) according to Claim 1 in which
A represents methylene, ethane-1,1-diyl, ethane-1,2-diyl, propane-1,1-diyl, propane-1,2-diyl, propane-1,3-diyl, propane-2,2-diyl, butane1,2-diyl, butane-1,3-diyl or butane-2,3-diyl,
Ar represents ortho-, meta- or para-phenylene, furandiyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl (in particular pyridine-2,3-diyl), pyrimidinediyl, pyridazinediyl, pyrazinediyl, 1,3,4-triazinediyl or 1,2,3-triazinediyl, each of which is optionally mono- or disubstituted by fluorine, chlorine, cyano, methyl, ethyl, cyclopropyl, trifluoromethyl, methoxy, ethoxy, methylthio, methylsulphinyl or methylsulphonyl,
G represents a single bond, methylene, ethane-1,2-diyl, -O-CH₂- or a grouping 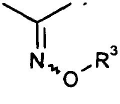 in which
R³ represents methyl, ethyl, n- or i-propyl,
R¹ represents hydrogen, cyano, methyl, ethyl or cyclopropyl,
R² represents hydrogen or methyl,
Y represents oxygen, sulphur or -NH- and
Z represents phenyl, 1,2,4-thiadiazolyl, 1,3,4-thiadiazolyl, 1,2,4-oxadiazolyl, 1,3,4-oxadiazolyl, pyridinyl, pyrimidyl, pyridazinyl, pyrazinyl, 1,2,3-triazinyl, 1,2,4-triazinyl, 1,3,5-triazinyl or 5,6-dihydro-1,4,2-dioxazinyl, each of which is optionally mono- to trisubstituted by identical or different substituents, where the possible substituents are preferably selected from the list below:
fluorine, chlorine, bromine, cyano, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl,
in each case doubly attached methylenedioxy, ethylenedioxy, each of which is optionally mono- to tetrasubstituted by identical or different substituents selected from the group consisting of fluorine, chlorine, methyl, trifluoromethyl and ethyl, a grouping 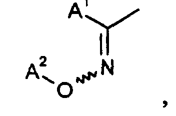
where
A¹ represents methyl, ethyl, n- or i-propyl, n-, i-, s- or t-butyl, cyclopropyl or cyclobutyl and
A² represents methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl.

3. Compounds of the formula (I) according to Claim 1,
in which
A represents methylene, ethane-1,1-diyl, ethane-1,2-diyl, propane-1,1-diyl, propane-1,2-diyl, propane-1,3-diyl, propane-2,2-diyl, butane-1,2-diyl, butane-1,3-diyl or butane-2,3-diyl,
Ar represents ortho-phenylene, pyridine-2,3-diyl or thiophene-2,3-diyl,
G represents a single bond, methylene, ethane-1,2-diyl, -O-CH₂- or a grouping 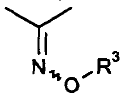 in which
R³ represents methyl,
R¹ represents methyl,
R² represents hydrogen,
Y represents oxygen, sulphur or -NH- and
Z represents phenyl, pyridyl, pyrimidyl, thienyl or 5,6-dihydro-1,4,2-dioxazinyl, each of which is optionally mono- to trisubstituted by identical or different substituents, where the possible substituents are preferably selected from those below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl,
in each case doubly attached methylenedioxy or ethylenedioxy, each of which is optionally mono- to tetrasubstituted by identical or different substituents selected from the group consisting of fluorine, chlorine, methyl, trifluoromethyl and ethyl or a grouping 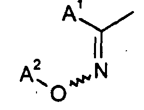 in which
A¹ represents methyl, ethyl, n- or i-propyl, n-, i-, s- or t-butyl, cyclopropyl or cyclobutyl and
A² represents methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl.

4. Pesticides, **characterized in that** they comprise at least one compound of the formula (I) according to Claim 1.

5. Method for controlling pests, **characterized in that** compounds of the formula (I) according to Claim 1 are allowed to act on pests and/or their habitat.

6. Process for preparing compounds of the formula (I) as defined in Claim 1, **characterized in that** oximes of the general formula (II) 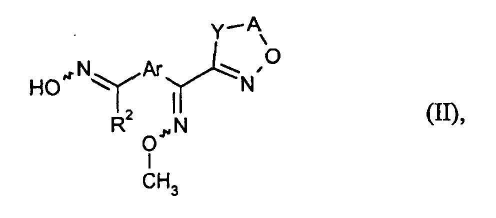 in which
A, Ar, Y and R² are as defined in Claim 1,
are reacted with an alkylating agent of the general formula (III) 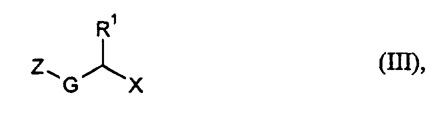 in which
G, R¹ and Z are as defined above and
X represents halogen, alkylsulphonyloxy or optionally substituted arylsulphonyloxy,
if appropriate in the presence of a diluent and if appropriate in the presence of an acid acceptor.

7. The use of compounds of the formula (I) according to Claims 1 to 3 for controlling pests.

8. Process for preparing pesticides, **characterized in that** compounds of the formula (I) according to Claims 1 to 3 are mixed with extenders and/or surfactants.

9. Compounds of the formula (II) 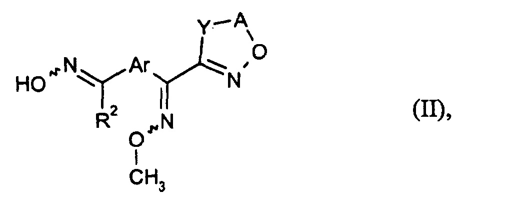 in which
R², Ar, Y and A are as defined in Claim 1.

10. Compounds of the formula (IV) 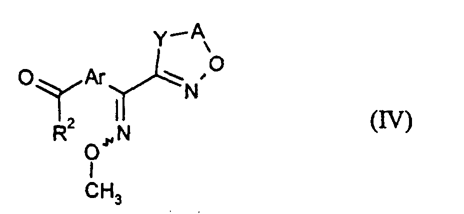 in which
R², Ar, Y and A are as defined in Claim 1.

## Revendications

1. Composés de formule (I) 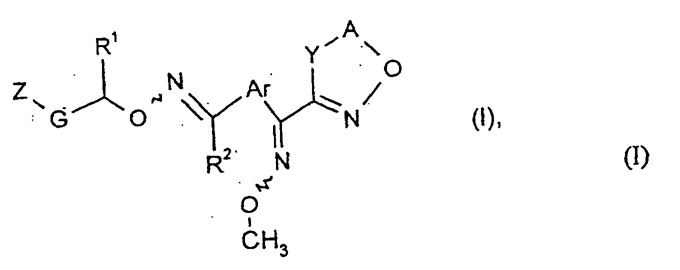 dans laquelle
A est un reste alcanediyle ayant 1 à 6 atomes de carbone,
Ar représente un reste phénylène ou un reste naphtylène, chacun éventuellement substitué, un reste hétéroarylène monocyclique ou bicyclique à noyau de 5 ou 6 chaînons dans chaque cas ou un reste hétéroarylène condensé au benzène à noyau de 5 ou 6 chaînons, dont l'un au moins dans chaque cas représente l'oxygène, le soufre ou l'azote et, le cas échéant, un ou deux autres représentent l'azote, les substituants possibles étant avantageusement choisis parmi ceux qui sont énumérés ci-après :
halogène, cyano, nitro, amino, hydroxy, formyle, carboxy, carbamoyle, thiocarbamoyle, alkyle, alkoxy, alkylthio, alkylsulfinyle ou alkylsulfonyle chacun linéaire ou ramifié ayant dans chaque cas 1 à 6 atomes de carbone, alcényle, alcényloxy ou alcynyloxy chacun linéaire ou ramifié ayant dans chaque cas 2 à 6 atomes de carbone, halogénalkyle, halogénalkoxy, halogénalkylthio, halogénalkylsulfinyle ou halogénalkylsulfonyle, chacun linéaire ou ramifié, ayant dans chaque cas 1 à 6 atomes de carbone et 1 à 13 atomes d'halogènes identiques ou différents, halogénalcényle ou halogénalcényloxy, chacun linéaire ou ramifié ayant dans chaque cas 2 à 6 atomes de carbone et 1 à 11 atomes d'halogènes identiques ou différents, alkylamino, dialkylamino, alkylcarbonyle, alkylcarbonyloxy, alkoxycarbonyle, alkylsulfonyloxy, hydroximinoalkyle ou alkoximinoalkyle chacun linéaire ou ramifié ayant dans chaque cas 1 à 6 atomes de carbone dans les parties alkyle individuelles, alkylène ou dioxyalkylène ayant chacun 1 à 6 atomes de carbone et chacun deux liaisons, chacun portant éventuellement un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
G représente une liaison simple, un reste alcanediyle éventuellement interrompu par un ou deux hétéroatomes, ayant 1 à 5 chaînons (toutefois l'atome de carbone qui porte R¹ est toujours lié à un atome de carbone de la chaîne alcanediyle) ou un groupement 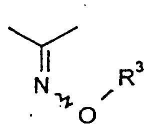 dans lequel
R³ est un reste alkyle ayant 1 à 4 atomes de carbone,
R¹ représente l'hydrogène, un groupe cyano, un reste alkyle, alkoxy, alkylthio, alkylamino ou dialkylamino ayant chacun 1 à 6 atomes de carbone dans les groupes alkyle et chacun portant éventuellement un substituant halogéno, cyano ou alkoxy en C₁ à C₄, ou un reste cycloalkyle de 3 à 6 atomes de carbone éventuellement substitué dans chaque cas par un halogène, un radical cyano, carboxy, alkyle en C₁ à C₄ ou (alkoxy en C₁ à C₄)-carbonyle,
R² est l'hydrogène ou un reste alkyle ayant 1 à 4 atomes de carbone,
Y représente l'oxygène, le soufre ou le groupe -NH- et
Z est un reste alkyle ayant 1 à 8 atomes de carbone éventuellement substitué une ou plusieurs fois identiques ou différentes par un halogène, un radical cyano, hydroxy, amino, alkoxy en C₁ à C₄, alkylthio en C₁ à C₄, alkylsulfinyle en C₁ à C₄ ou alkylsulfonyle en C₁ à C₄ (dont chacun peut éventuellement être substitué par un halogène) ;
un reste alcényle ou alcynyle ayant chacun jusqu'à 8 atomes de carbone et chacun étant éventuellement substitué par un halogène ;
un reste cycloalkyle de 3 à 6 atomes de carbone portant éventuellement un ou plusieurs substituants, identiques ou différents, halogéno, cyano, carboxy, phényle (qui est éventuellement substitué par un radical halogéno, cyano, alkyle en C₁ à C₄, halogénalkyle en C₁ à C₄, alkoxy en C₁ à C₄ ou halogénalkoxy en C₁ à C₄) alkyle en C₁ à C₄ ou (alkoxy en C₁ à C₄)-carbonyle ;
ou bien un reste, portant éventuellement dans chaque cas un ou plusieurs substituants identiques ou différents, phényle, naphtyle ou hétérocyclyle à noyau de 3 à 7 chaînons dont l'un au moins est l'oxygène, le soufre ou l'azote et le cas échéant, un ou deux autres représentent l'azote, les substituants possibles étant avantageusement choisis parmi ceux qui sont énumérés ci-après :
halogéno, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant dans chaque cas 1 à 6 atomes de carbone ;
alcényle ou alcényloxy chacun linéaire ou ramifié ayant dans chaque cas 2 à 6 atomes de carbone ; halogénalkyle, halogénalkoxy, halogénalkylthio, halogénalkylsulfinyle ou halogénalkylsulfonyle chacun linéaire ou ramifié ayant dans chaque cas 1 à 6 atomes de carbone et 1 à 13 atomes d'halogènes identiques ou différents ; halogénalcényle ou halogénalcényloxy chacun linéaire ou ramifié ayant dans chaque cas 2 à 6 atomes de carbone et 1 à 11 atomes d'halogènes identiques ou différents ;
alkylamino, dialkylamino, alkylcarbonyle alkylcarbonyloxy, alkoxycarbonyle ou alkylsulfonyloxy chacun linéaire ou ramifié ayant dans chaque cas 1 à 6 atomes de carbone dans les parties alkyle individuelles ;
alkylène ou dioxyalkylène ayant chacun 1 à 6 atomes de carbone et chacun deux liaisons, portant chacun le cas échéant 1 ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
hétérocyclyle ou hétérocyclylméthyle ayant chacun dans le noyau 3 à 7 chaînons dont 1 à 3 sont dans chaque cas des hétéroatomes identiques ou différents - notamment azote, oxygène et/ou soufre -
ou un groupement 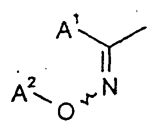 dans lequel
A¹ est un radical alkyle ayant 1 à 4 atomes de carbone ou cycloalkyle ayant 1 à 6 atomes de carbone et
A² est un radical alkyle ayant 1 à 4 atomes de carbone éventuellement substitués par un groupe cyano, alkoxy, alkylthio, alkylamino, dialkylamino ou phényle, un radical alcényle ou un radical alcynyle ayant chacun 2 à 4 atomes de carbone.

2. Composés de formule (I) suivant la revendication 1, formule dans laquelle
A est un reste méthylène, éthane-1,1-diyle, éthane-1,2-diyle, propane-1,1-diyle, propane-1,2-diyle, propane-1,3-diyle, propane-2,2-diyle butane-1,2-diyle, butane-1,3-diyle ou butane-2,3-diyle,
Ar est un reste ortho-, méta- ou paraphénylène furannediyle, thiophènediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle (en particulier pyridine-2,3-diyle), pyrimidinediyle, pyridazinediyle, pyrazinediyle, 1,3,4-triazinediyle ou 1,2,3-triazinediyle portant éventuellement dans chaque cas 1 ou 2 substituants fluoro, chloro, cyano, méthyle, éthyle, cyclopropyle, trifluorométhyle, méthoxy, éthoxy, méthylthio, méthylsulfinyle ou méthylsulfonyle,
G est une liaison simple, un reste méthylène, éthane-1,2-diyle, -O-CH₂- ou un groupement 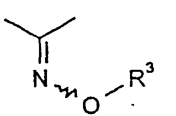 dans lequel
R³ est un radical méthyle, éthyle, n-propyle ou isopropyle,
R¹ est l'hydrogène, un groupe cyano, un reste méthyle, éthyle ou cyclopropyle,
R² est l'hydrogène ou un reste méthyle,
Y est l'oxygène, le soufre ou un groupe -NH- et
Z représente un reste phényle, 1,2,4-thiadiazolyle, 1,3,4-thiadiazolyle, 1,2,4-oxadiazolyle, 1,3,4-oxadiazolyle, pyridinyle, pyrimidyle, pyridazinyle, pyrazinyle, 1,2,3-triazinyle, 1,2,4-triazinyle, 1,3,5-triazinyle ou 5,6-dihydro-1,4,2-dioxazinyle portant éventuellement un à trois substituants identiques ou différents, les substituants possibles étant avantageusement choisis parmi ceux qui sont énumérés ci-après : fluor, chlore, brome, cyano, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluoro-méthylthio, trifluorométhylthio, difluorochlorométhylthio, trifluorométhylsulfinyle, trifluorométhylsulfonyle, méthoxycarbonyle, éthoxycarbonyle,
méthylènedioxy ou éthylènedioxy ayant chacun deux liaisons et portant chacun, le cas échéant un à . quatre substituants, identiques ou différents, fluoro, chloro, méthyle, trifluorométhyle ou éthyle, ou un groupement 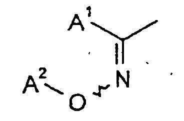 dans lequel
A¹ est un radical méthyle, éthyle, n-propyle, isopropyle, n-butyle, isobutyle, sec.- butyle, tertiobutyle, cyclopropyle ou cyclobutyle et
A² est un radical méthyle, éthyle, n-propyle, isopropyle, n-butyle, isobutyle, sec.- butyle, tertiobutyle, allyle, propargyle, but-2-ène-1-yle, 2-méthyl-prop-1-ène-3-yle, cyanométhyle, méthoxyméthyle, éthoxyméthyle, méthoxyéthyle, éthoxyéthyle, méthylthiométhyle, éthylthio-méthyle, méthylthio-éthyle, éthylthioéthyle, diméthylaminométhyle, diméthylaminoéthyle, méthylaminométhyle, méthylaminoéthyle ou benzyle.

3. Composés de formule (I) suivant la revendication 1, formule dans laquelle
A est un reste méthylène, éthane-1,1-diyle, éthane-1,2-diyle, propane-1,1-diyle, propane-1,2-diyle, propane-1,3-diyle, propane-2,2-diyle, butane-1,2-diyle, butane-1,3-diyle ou butane-2,3-diyle,
Ar est un reste orthophénylène, pyridine-2,3-diyle ou thiophène-2,3-diyle,
G est une liaison simple, un groupe méthylène, éthane-1,2-diyle, -O-CH₂- ou un groupement 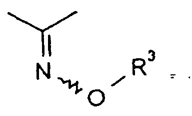 dans lequel
R³ est un radical méthyle,
R¹ est un radical méthyle,
R² est l'hydrogène,
Y est l'oxygène, le soufre ou un groupe -NH- et
Z représente un reste phényle, pyridyle, pyrimidyle, thiényle ou 5,6-dihydro-1,4,2-dioxazinyle portant chacun le cas échéant un à trois substituants identiques ou différents, les substituants possibles étant avantageusement choisis parmi ceux qui sont énumérés ci-après : fluor, chlore, brome, cyano, méthyle, éthyle, n-propyle, isopropyle, n-butyle, isobutyle, sec.- butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, ou éthylsulfonyle, trifluorométhyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle, méthylènedioxy ou éthylènedioxy ayant chacun deux liaisons et portant chacun le cas échéant un à quatre substituants, identiques ou différents, fluoro, chloro, méthyle, trifluorométhyle ou éthyle, ou un groupement 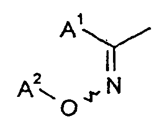 dans lequel
A¹ est un radical méthyle, éthyle, n-propyle, isopropyle, n-butyle, isobutyle, sec.- butyle, tertiobutyle, cyclopropyle ou cyclobutyle, et
A² est un radical méthyle, éthyle, n-propyle, isopropyle, n-butyle, isobutyle, sec.- butyle, tertiobùtyle, allyle, propargyle, but-2-ène-1-yle, 2-méthylprop-1-ène-3-yle, cyanométhyle, méthoxyméthyle, éthoxyméthyle, méthoxyéthyle, éthoxyéthyle, méthylthiométhyle, éthylthiométhyle, méthylthioéthyle, éthylthioéthyle, diméthylaminométhyle, diméthylaminoéthyle, méthylaminométhyle, méthylaminoéthyle ou benzyle.

4. Composition pesticide, **caractérisée par** une teneur en au moins un composé de formule (I) suivant la revendication 1.

5. Procédé pour combattre des parasites, **caractérisé en ce qu'**on fait agir des composés de formule (I) suivant la revendication 1 sur les parasites et/ou sur leur milieu.

6. Procédé de production de composés de formule (I) telle que définie dans la revendication 1,
**caractérisé en ce qu'**on fait réagir des oximes de formule générale (II) 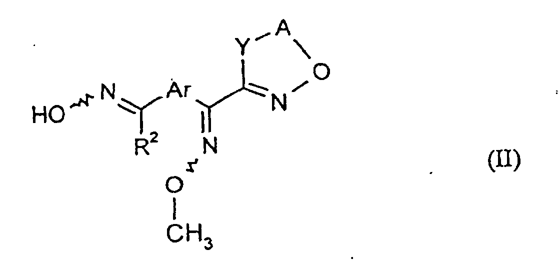 dans laquelle
A, Ar, Y et R² ont les définitions indiquées dans la revendication 1,
avec un réactif d'alkylation de formule générale (III), 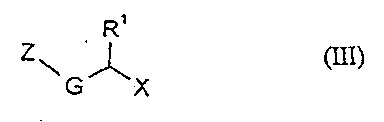 dans laquelle
G, R¹ et Z ont les définitions indiquées ci-dessus et X est un halogène, un radical alkylsulfonyloxy ou un radical arylsulfonyloxy éventuellement substitué,
le cas échéant en présence d'un diluant et en présence éventuelle d'un accepteur d'acide.

7. Utilisation de composés de formule (I) suivant les revendications 1 à 3 pour combattre des parasites.

8. Procédé de préparation de compositions pesticides, **caractérisé en ce qu'**on mélange des composés de formule (I) suivant les revendications 1 à 3 avec des diluants et/ou des agents tensioactifs.

9. Composés de formule (II), 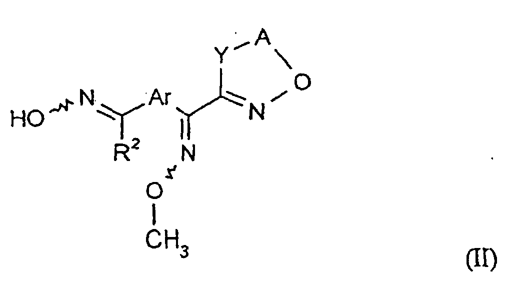 dans laquelle
R², Ar, Y et A ont les définitions indiquées dans la revendication 1.

10. Composés de formule (IV) 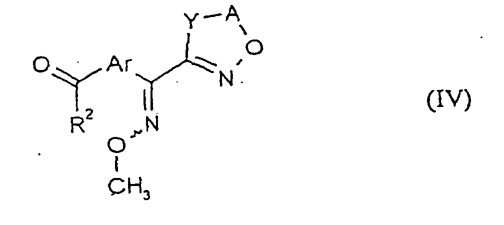 dans laquelle
R², Ar, Y et A ont les définitions indiquées dans la revendication 1.
